(19) Europäisches Patentamt
European Patent Office
Office européen des brevets

(11) EP 1 655 282 A1

(12) # EUROPEAN PATENT APPLICATION

(43) Date of publication:
**10.05.2006 Bulletin 2006/19**

(51) Int Cl.:
***C07C 243/38*** *(2006.01)* ***C08K 5/25*** *(2006.01)*

(21) Application number: **05024307.0**

(22) Date of filing: **08.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **08.11.2004 JP 2004323773**

(71) Applicant: **Kabushiki Kaisha Ueno Seiyaku Oyo Kenkyujo**
**Osaka-shi,**
**Osaka-fu (JP)**

(72) Inventor: **Wakamori, Hiroyuki**
**Tanba-shi**
**Hyogo-ken (JP)**

(74) Representative: **Albrecht, Thomas et al**
**Kraus & Weisert,**
**Thomas-Wimmer-Ring 15**
**80539 München (DE)**

(54) **Hydroxynaphthoic acid hydrazide compounds and method for preparing the same**

(57) Provided is a novel hydroxynaphthoic acid hydrazide compound represented by the formulae [1] or [2]:

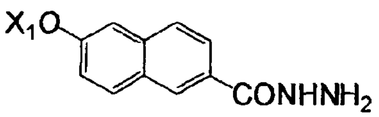
[1]

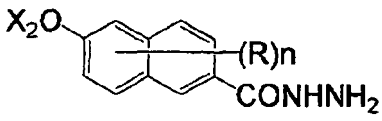
[2]

and a method for preparing the same.



Printed by Jouve, 75001 PARIS (FR)

## Description

BACKGROUND OF THE INVENTION

FIELD OF THE INVENTION

This application relates to a novel hydroxynaphthoic acid hydrazide compound and a method for preparing the same.

ART RELATED

Among hydroxynaphthoic acids, 2-hydroxy-3-naphthoic acid and 2-hydroxy-6-naphthoic acid are well known and widely used in various field as an intermediate for synthesizing organic dyes and synthetic resins.

Hydrazide of 2-hydroxy-3-naphthoic acid or a derivative thereof is well known and used in various fields. For example it is used as an intermediate for synthesizing a coupler for azo compounds, which is used as a electron generating material for photoreceptor in the electrophotography system (Japanese Patent Application Laid Open No. 6-095403), as an additive for rubber composition which is used for manufacturing tires or as an intermediate for synthesizing said additive (USP 5,534,569 and Japanese Patent Application Laid Open No, 11-292834), and as epoxy curing agent or curing accelerator for epoxy resins (Japanese Patent Application Laid Open No. 9-67466).

In contrast, 2-hydroxy-6-naphthoic acid hydrazide has not been known. An example of known hydrazide of a 2-hydroxy-6-naphthoic acid derivative is 2-methoxy-6-naphthoic hydrazide, which is white crystal obtained by reacting 2-methoxy-6-naphthoic acid chloride (6-methoxy-2-naphthylcarbonyl chloride) and hydrazine, and is used as an intermediate for manufacturing fluorescent compound (Japanese Patent Application Laid Open No. 2001-271062.

SUMMARY OF THE INVENTION

An object of the present invention is to provide a novel hydroxynaphthoic acid hydrazide compound as well as a method for preparing the same.

In one aspect of the present invention, a hydroxynaphthoic acid hydrazide compound represented by formula [1]:

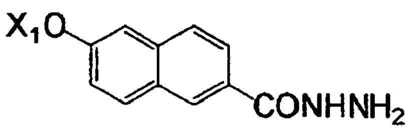

[1]

wherein, $X_1$ is hydrogen, C2-6 alkyl which may be substituted by hydroxy and/or halogen, C7-11 aralkyl or alkali metal, or a salt thereof is provided.

The present invention further provides a method for preparing the hydroxynaphthoic acid compound of formula [1] which comprises the step of:

reacting an alkyl ester of hydroxynaphthoic acid compound represented by formula [3]:

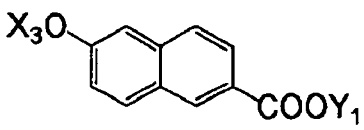

[3]

wherein, $Y_1$ is C1-6 alkyl; $X_3$ is hydrogen, C2-6 alkyl which may be substituted by hydroxy and/or halogen, or C7-11 aralkyl,
with at least one hydrazine compound selected from the group consisting of hydrazine monohydrate, hydrazine sulfate, dihydrazine sulfate, hydrazine monohydrochloride, hydrazine dihydrochloride, and hydrazine monohydrobromide.

In another aspect of the present invention, a hydroxynaphthoic acid hydrazide compound resented by formula

[2]:

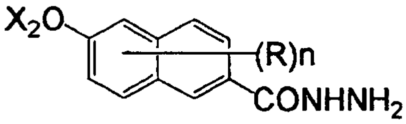

[2]

wherein, $X_2$ is hydrogen, C1-6 alkyl which may be substituted by hydroxy and/or halogen, C7-11 aralkyl or alkali metal; R is C1-6 alkyl, C1-6 alkoxy, halogen, nitro, or hydroxy; n is an integer of 1-6, in which when n is an integer of 2-6, the Rs may be the same or different, or a salt thereof is provided.

According to the present invention, a method for preparing the hydroxynaphthoic acid hydrazide compound of formula [2], which comprises the step of:

reacting an alkyl ester of hydroxynaphthoic acid compound represented by formula [4]:

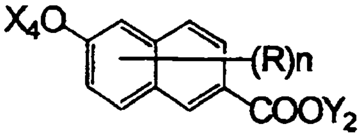

[4]

wherein $Y_2$ is C1-6 alkyl; $X_4$ is hydrogen, C1-6 alkyl which may be substituted by hydroxy and/or halogen, or C7-11 aralkyl; R is C1-6 alkyl, C1-6 alkoxy, halogen, nitro, or hydroxy; n is an integer of 1-6, wherein n is 2-6, the Rs may be the same or different,

with at least one hydrazine compound selected from the group consisting of hydrazine monohydrate, hydrazine sulfate, dihydrazine sulfate, hydrazine monohydrochloride, hydrazine dihydrochloride, and hydrazine monohydrobromide is provided.

BRIEF DESCRIPTION OF THE DRAWINGS

Figure 1 represents an infrared absorption spectrum (KBr) of the hydroxynaphthoic acid hydrazide obtained in Example 1.

Figure 2 represents an infrared absorption spectrum (KBr) of the 1-bromo-2-hydroxy-6-naphthoic acid hydrazide obtained in Example 2.

Figure 3 represents an infrared absorption spectrum (KBr) of the 2-(2'-hydroxyethyl)oxy-6-naphthoic acid hydrazide obtained in Example 3.

Figure 4 represents an infrared absorption spectrum (KBr) of the 2-hydroxy-6-naphthoic acid hydrazide hydrochloride obtained in Example 4.

DETAILED DESCRIPTION OF THE PREFERRED EMBODIMENT

According to the 1st embodiment of the present invention, 2-hydroxy-6-naphthoic hydrazide compound of formula [1], wherein $X_1$ is hydrogen, C2-6 alkyl which may be substituted by hydroxy and/or halogen, C7-11 aralkyl or alkali metal is provided.

Examples of the C2-6 alkyl which may be substituted by hydroxy and/or halogen of $X_1$ may include ethyl, n-propyl, i-propyl, n-butyl, i-butyl, t-butyl, n-pentyl, n-hexyl, 2-hydroxyethyl, 2-chloroethyl and 4-hydroxybutyl. Examples of the C7-11 aralkyl may include benzyl, phenethyl, phenylpropyl, 1-naphthylmethyl and 2-naphthylmethyl. Examples of alkali metals may include sodium, potassium and lithium.

In the method of the present invention for preparing the compound of formula [1], the starting material, i.e. the alkyl ester of hydroxynaphthoic acid compound of formula [3] may be prepared by means of any known method. For example, the ester may be prepared according to scheme 1 shown below:

Scheme 1

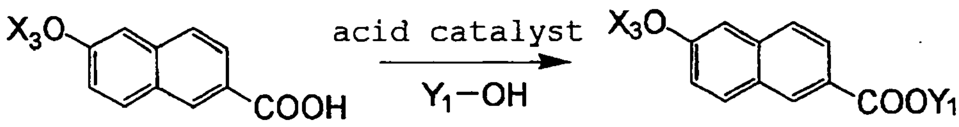


[5] [3]

wherein $X_3$ in formula [5] is hydrogen, C2-6 alkyl which may be substituted by hydroxy and/or halogen, or C7-11 aralkyl.

In more detail, an esterification reaction between the 2-hydroxy-6-naphthoic acid compound of formula [5] and a C1-6 alcohol ($Y_1$-OH) may be conducted according to a known manner in the presence of an acid catalyst such as sulfuric acid, p-toluene sulfonic acid, or an acidic ion-exchange resin. When $X_3$ of formula [5] is hydrogen, i.e. the compound of formula [5] is 2-hydroxy-6-naphthoic acid, said starting compound may be obtained by reacting dehydrated potassium β-naphthol in an inert medium with carbon dioxide under pressure and heating to give a solution, and precipitating the desired compound by adding acid. The obtained compound may be purified, if necessary.

The compound of formula [3] or [5] wherein $X_3$ is C2-6 alkyl which may be substituted hydroxy and/or halogen, or C7-11 aralkyl, may be prepared by reacting 2-hydroxy-6-naphthoic acid or an ester thereof, which is obtainable by the above step, and halogenated alkyl or halogenated aralkyl which corresponds to the $X_3$ moiety in an solvent such as N,N-dimethylformamide in the presence of a basic compound such as potassium carbonate.

According to the 2nd embodiment of the present invention, a hydroxynaphthoic acid hydrazide compound of formula [2] or a salt thereof is provided. In the formula [2], $X_2$ is hydrogen, C1-6 alkyl which may be substituted by hydroxy and/or halogen, C7-11 aralkyl or alkali metal; R is C1-6 alkyl, C1-6 alkoxy, halogen, nitro, or hydroxy; n is an integer of 1-6, in which when n is an integer of 2-6, the Rs may be the same or different.

Examples of C1-6 alkyl which may be substituted by hydroxy and/or halogen of $X_2$ may include methyl, ethyl, n-propyl, i-propyl, n-butyl, i-butyl, t-butyl, n-pentyl, i-propyl, n-butyl, i-butyl, t-butyl, n-pentyl, n-hexyl, 2-hydroxyethyl, 2-chloroethyl and 4-hydroxybutyl. Examples of C7-11 aralkyl group may include benzyl, phenethyl, phenylpropyl, 1-naphthylmethyl and 2-naphthylmethyl. Examples of alkali metals may include sodium, potassium and lithium.

The "R", which represents the substituent on the naphthalene ring in formula [2], is C1-6 alkyl, C1-6 alkoxy, halogen, nitro or hydroxy.

The "n", which represents the number of the substituents on the naphthalene ring, is an integer of 1-6. When n is 2-6, i.e. there are two or more substituents "R" on the naphthalene ring, the Rs may be the same or different.

According to the method of the present invention for preparing the compound of formula [2], compound of formula [4] is reacted with at least one hydrazine compound.

According to the method, the starting material, i.e. the alkyl ester of hydroxynaphthoic acid compound of formula [4] may be prepared by means of any known method. For example, the ester of formula [4] may be prepared in the same manner as that of formula [3] according to scheme 2 shown below:

[Scheme 2]

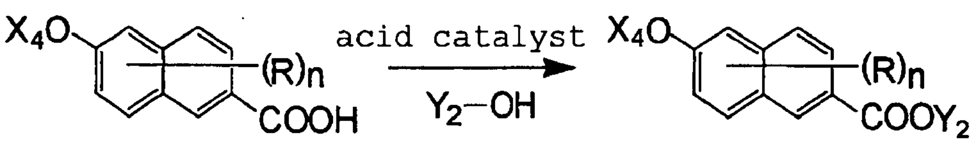


[6] [4]

wherein $X_4$ in formula [6] is hydrogen, C1-6 alkyl which may be substituted by hydroxy and/or halogen or C7-11 aralkyl; R is C1-6 alkyl, C1-6 alkoxy, halogen, nitro or hydroxy; n is an integer of 1-6, in which when n is an integer of 2-6, the Rs may be the same or different.

Alternatively, the compound of formula [4] may be prepared from hydroxynaphthoic acid compound represented by formula [7], which does not have substituents on the naphthalene ring, according to scheme 3 below. In the process,

an alkyl ester of formula [8] is prepared from the acid compound of formula [7] and then, the substituent(s) is introduced on to the naphthalene ring to give the compound of formula [4].

[Scheme 3]

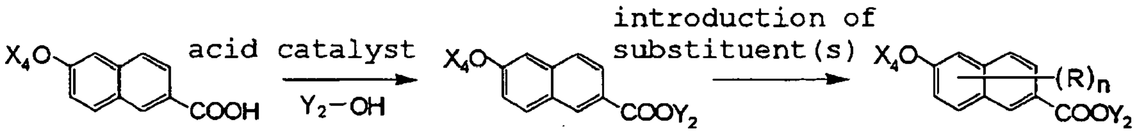


[7] [8] [4]

wherein in the compounds of formulae [7] and [8], $X_4$ is hydrogen, C1-6 alkyl which may be substituted by hydroxy and/or halogen or C7-11 aralkyl; $Y_2$ represents C1-6 alkyl.

According to the present invention, the hydrazide compound of formulae [1] and [2] may be obtained by reacting thus obtained hydroxynaphthoic acid compound or an ester thereof of formulae [3] and [4] respectively with at least one hydrazine compound selected from the group consisting of hydrazine monohydrate, hydrazine sulfate, dihydrazine sulfate, hydrazine monohydrochloride, hydrazine dihydrochloride, and hydrazine monohydrobromide.

Among the hydrazine compounds, hydrazine monohydrate is preferably used, since the reaction generates water as bi-product in addition to the desired hydrazide and the mixture of hydrazide and water can easily be handled.

According to the present invention, the amount of the hydrazine compound used in the method may be 1.2-5.0 mole, preferably 2.0-2.5 mole per 1 mole of the alkyl ester of hydroxynaphthoic compound of formula [3] or [4].

The reaction between the compound of formula [3] or [4] and the hydrazine compound is preferably conducted at a temperature of 20-100°C, and especially at 80-100°C, in order to facilitate the reaction and reduce the generation of bi-products.

The solvent used in the hydrazide forming reaction may be any solvents as long as the solvent is inert against the hydrazide forming reaction and alcohols such as methanol, ethanol, n-propanol, n-butanol and 2-ethylhexyl alcohol may preferably be used.

The hydrazide forming reaction may be conducted until at least 80 mol%, preferably 90mol% and more preferably 95% of the starting compound of formula [3] or [4] is converted into the corresponding hydrazide. The conversion of the starting material may be confirmed by means of high performance liquid chromatography or the like.

The reaction time may vary depending on the reaction temperature, solvent and the like and in general, it takes 5-50 hours. The hydrazide forming reaction may be carried out in either of a batch-wise or continuous manner.

After the hydrazide forming reaction is completed, the reaction mixture may be cooled, concentrated or added with a poor solvent such as water so that the 2-hydroxy-6-naphthoic acid hydrazide compound are precipitated. The precipitates may be collected from the reaction mixture by means of a known method such as centrifugation or filter press and dried.

Thus obtained 2-hydroxy-6-naphthoic acid hydrazide compound of formula [1] or [2] may be purified, if necessary, by means of re-crystallizing the same or washing the same with an organic solvent and/or water. In order to prepare 2-hydroxy-6-naphthoic acid hydrazide compound of formula [2] from thus obtained 2-hydroxy-6-naphthoic acid hydrazide compound having no substituent on the naphthalene ring, a substituent selected from the group consisting of C1-6 alkyl, C1-6 alkoxy, halogen, nitro and hydroxy is introduced in a conventional manner to the non-substituted compound. An alkali metal salt of 2-hydroxy-6-naphthoic acid hydrazide compound of formula [1] or [2], that is, the compound of formula [1] or [2] wherein $X_1$ or $X_2$ is an alkali metal, may be prepared by reacting the compound of formula [1] or [2] wherein $X_1$ or $X_2$ is hydrogen, with a basic alkali metal compound, such as alkali metal oxide and alkali metal alkoxide, in water and/or organic solvent.

Further, the salt of the 2-hydroxy-6-naphthoic acid hydrazide compound of formula [1] or [2] such as hydrochloride, sulfate, nitrate, benzene sulfonate and p-toluenesulfonate may be obtained by reacting 2-hydroxy-6-naphthoic acid hydrazide compound of formula [1] or [2] wherein $X_1$ or $X_2$ is not alkali metal, with an acid selected from the group consisting of hydrochloric acid, sulfuric acid, nitric acid, benzenesulfonic acid and p-toluenesulfonic acid. The desired salt may be obtained by precipitating by means of concentrating or evaporating the solvent and collecting the same from the reaction mixture.

The 2-hydroxy-6-naphthoic acid hydrazide compound or a salt thereof may be used as a coupler component for manufacturing an azo compound, as an additive for rubber composition which is used for manufacturing tires or as an intermediate for synthesizing the additive and as epoxy curing agent or curing accelerator for epoxy resins.

Examples

The present invention is further illustrated with reference to the following examples. However, it is to be understood that the invention is not intended to be limited to the specific examples.

Example 1

A 2-hydroxy-6-naphthoic acid hydrazide compound of formula [I] was prepared.

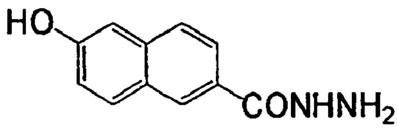

[I]

Methyl 2-hydroxy-6-naphthoate 25.3g (125mmol), which was obtained by a known method, was dispersed in n-butanol 100g, and hydrazine monohydrate 17.3g(275mmol) was added dropwise thereto at room temperature. When the addition of the hydrazine monohydrate was terminated, the mixture in the reaction vessel was homogeneous solution.
The reaction mixture was heated from room temperature to 100°C over 1 hour and kept at the temperature with stirring to conduct the hydrazide forming reaction. After reacted at the temperature for 48 hours, the reaction mixture was analyzed with high performance liquid chromatography and found that 95mol% of the starting 2-hydroxy-6-naphthoic acid was converted into hydrazide and terminated the reaction by cooling the reaction to room temperature. The precipitates were collected from the reaction by suction filtration.
The precipitates were dispersed in cold methanol 80g and then, washed, filtered and dried to give 20.3g (100.4mmol) of 6-hydroxy-2-naphthoic acid hydrazide, represented by formula [I], as white crystal.

MS: m/z(-)201, m/z(+)203 (MW 202.2).

Decomposition point 257°C
$^1$H-NMR (DMSO-$d_6$, 400MHz):
10.03(1H,s), 9.80(1H,s), 8.30(1H,d, J=1.7Hz), 7.85(1H,d,J=8.6Hz ),7.80(1H,dd,J=1.7,8.6Hz),7.72(1H,d,J=8.6Hz),7.15 (1H,s),7.1 4(1H,dd,J=1.7,8.6Hz),4.51(2H,s).
The infrared absorption spectrum (KBr) of thus obtained 2-hydroxy-6-naphthoic acid hydrazide is shown in Figure 1.

Example 2

A 2-hydroxy-6-naphthoic acid hydrazide compound of formula [II]:

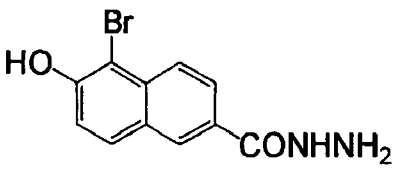

[II]

was prepared in the same manner as example 1 except for using methyl 1-bromo-2-hydroxy-6-naphthoate 35.1g (125mmol) instead of methyl 2-hydroxy-6-naphthoate. 28.9g (102.8mmol) of 1-bromo-2-hydroxy-6-naphthoic acid hydrazide (formula [II]) was obtained as white crystal.
MS: m/z(-)279,281,m/z(+)281,283(MW 281.1).
Decomposition point:223°C.
The infrared absorption spectrum (KBr) of thus obtained 1-bromo-2-hydroxy-6-naphthoic acid hydrazide is shown in Figure 2.

Example 3

A 2-hydroxy-6-naphthoic acid hydrazide compound of formula [III]:

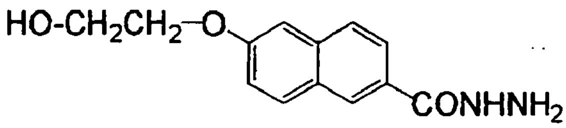

(III)

was prepared by the same manner as example 1, except for using methyl 2-(2'-hydroxyethyl)oxy-6-naphthoate 30.8g (125mmol) instead of methyl 2-hydroxy-6-naphthoate. 23.6g(95.8mmol) of 2-(2'-hydroxyethyl)oxy-6-naphthoic acid hydrazide (formula [III]) was obtained as white crystal.
MS:m/z (-) 245 m/z (+) 247 (MW 246.3).

Decomposition point: 176°C

The infrared absorption spectrum (KBr) of thus obtained 2-(2'-hydroxyethyl)oxy-6-naphthoic acid hydrazide is shown in Figure 3.

Example 4

A 2-hydroxy-6-naphthoic acid hydrazide compound of formula [IV] was prepared.

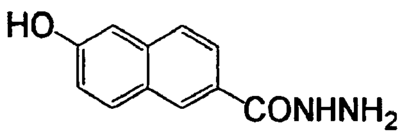

, HCl (IV)

2-hydroxy-6-naphthoic acid hydrazide 1.0g (4.9mmol) obtained in Example 1 was dispersed in methanol 20g and 36 wt% of aqueous hydrochloric acid 0.5g (4.9mmol) was added thereto. After the addition of the hydrochloric acid, 2-hydroxy-6-naphthoic acid hydrazide was converted to hydrochloride of 2-hydroxy-6-naphthoic acid hydrazide and dissolved. Then, methanol was evaporated and the residue was dried at 80°C to give 1.2g (4.9mmol) of hydrochloric acid salt of 2-hydroxy-6-naphthoic acid hydrazide.
The infrared absorption spectrum (KBr) of thus obtained hydrochloride of 2-hydroxy-6-naphthoic acid hydrazide is shown in Figure 4.

## Claims

1. A hydroxynaphthoic acid hydrazide compound represented by formula [1]:

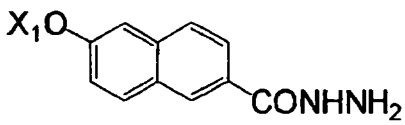

[1]

wherein, $X_1$ is hydrogen, C2-6 alkyl which may be substituted by hydroxy and/or halogen, C7-11 aralkyl or alkali metal, or a salt thereof.

2. A hydroxynaphthoic acid hydrazide compound represented by formula [2]:

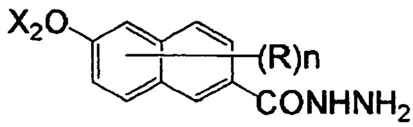

[2]

wherein, $X_2$ is hydrogen, C1-6 alkyl which may be substituted by hydroxy and/or halogen, C7-11 aralkyl or alkali metal; R is C1-6 alkyl, C1-6 alkoxy, halogen, nitro, or hydroxy; n is an integer of 1-6, in which when n is an integer of 2-6, the Rs may be the same or different,
or a salt thereof.

**3.** The salt of hydroxynaphthoic acid hydrazide compound according to claim 1 or 2, wherein the salt is selected from the group consisting of hydrochloride, sulfate, benzene sulfonate and p-toluenesulfonate, provided that $X_1$ or $X_2$ is not alkali metal.

**4.** A method for preparing hydroxynaphthoic acid hydrazide compound of formula [1]:

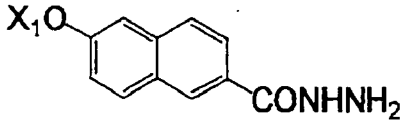

[1]

wherein $X_1$ is the same as defined in claim 1, which comprises the step of:

reacting an alkyl ester of hydroxynaphthoic compound represented by formula [3]:

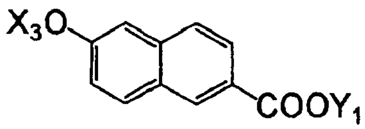

[3]

wherein, $Y_1$ is C1-6 alkyl; $X_3$ is hydrogen, C2-6 alkyl which may be substituted by hydroxy and/or halogen, or C7-11 aralkyl,
with at least one hydrazine compound selected from the group consisting of hydrazine monohydrate, hydrazine sulfate, dihydrazine sulfate, hydrazine monohydrochloride, hydrazine dihydrochloride, and hydrazine monohydrobromide.

**5.** A method for preparing a hydroxynaphthoic acid hydrazide compound represented by formula [2]:

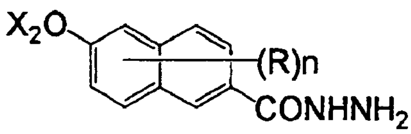

[2]

wherein, $X_2$, R and n are the same as defined in Claim 2,
which comprises the step of:

reacting an alkyl ester of hydroxynaphthoic compound represented by formula [4]:

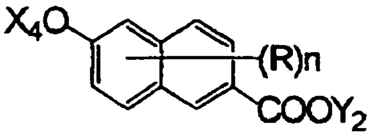

[4]

wherein $Y_2$ is C1-6 alkyl; $X_4$ is hydrogen, C1-6 alkyl which may be substituted by hydroxy and/or halogen, or C7-11 aralkyl; R is C1-6 alkyl, C1-6 alkoxy, halogen, nitro, or hydroxy; n is an integer of 1-6, wherein n is 2-6, the Rs may be the same or different,
with at least one hydrazine compound selected from the group consisting of hydrazine monohydrate, hydrazine sulfate, dihydrazine sulfate, hydrazine monohydrochloride, hydrazine dihydrochloride, and hydrazine monohydrobromide.

**6.** The method of Claim 4 or 5, wherein the reaction is conducted at a temperature of 20-100°C.

**7.** The method of any one of Claims 4, 5 and 6, wherein the hydrazine compound is hydrazine monohydrate.

Figure 1

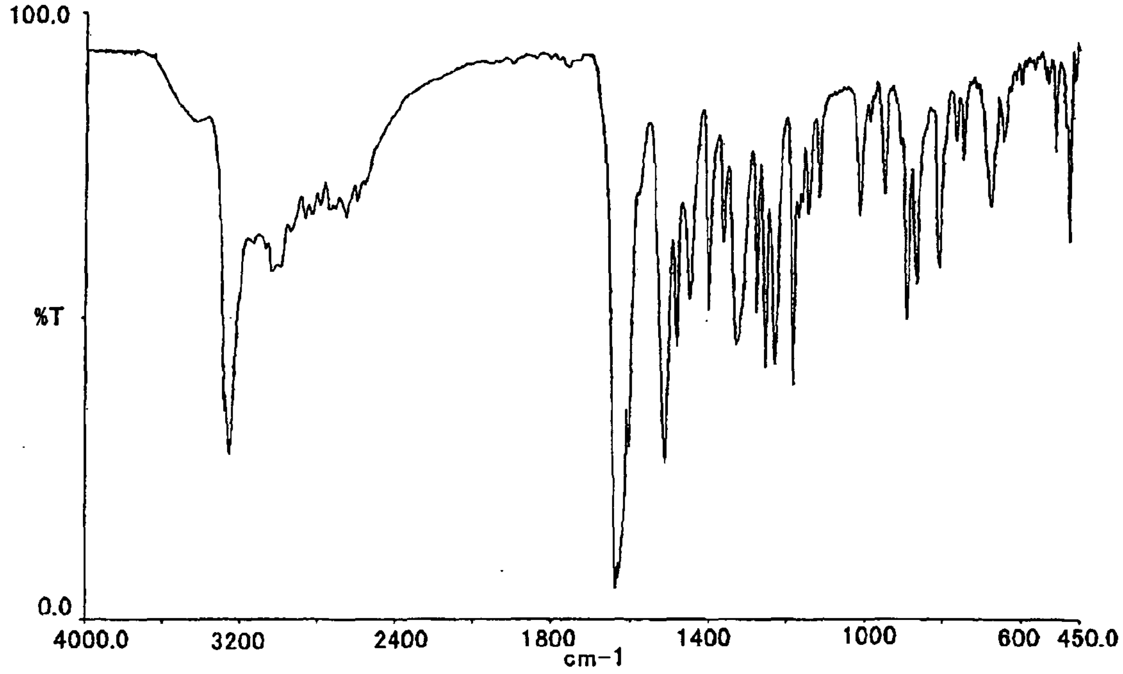

Figure 2

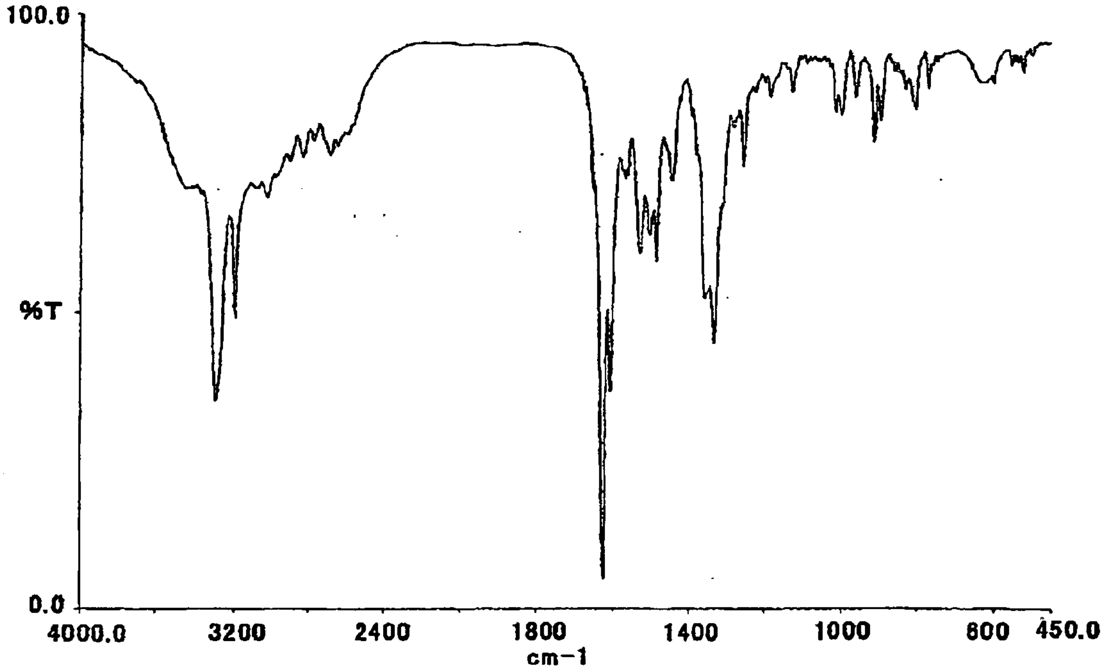

Figure 3

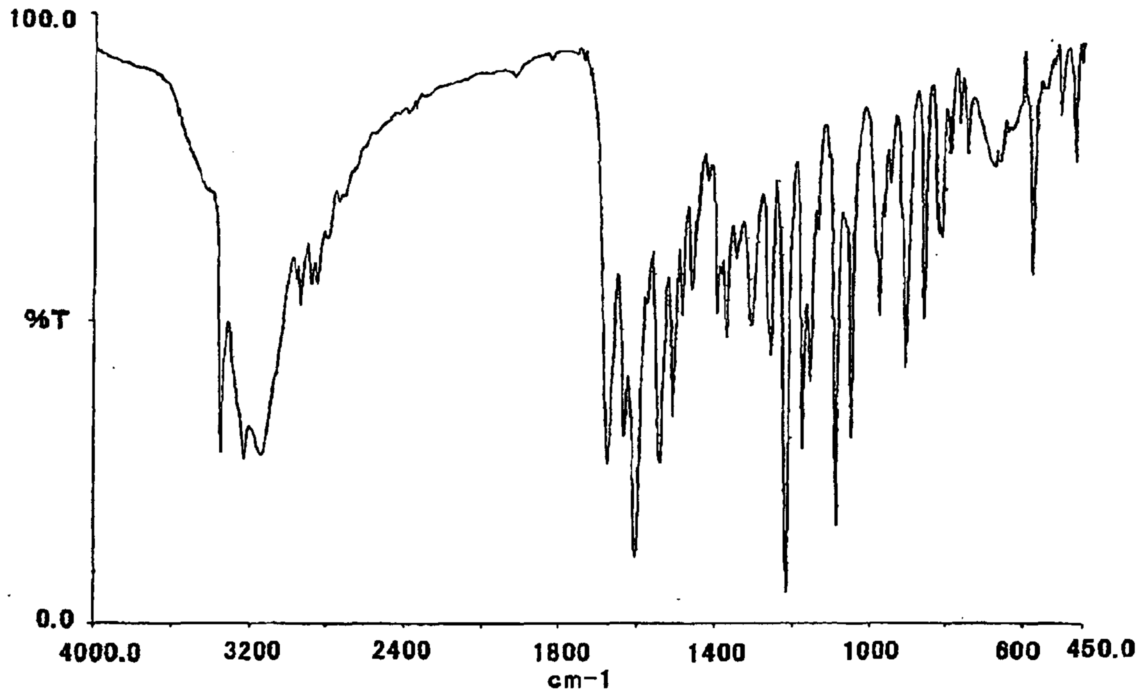

Figure 4

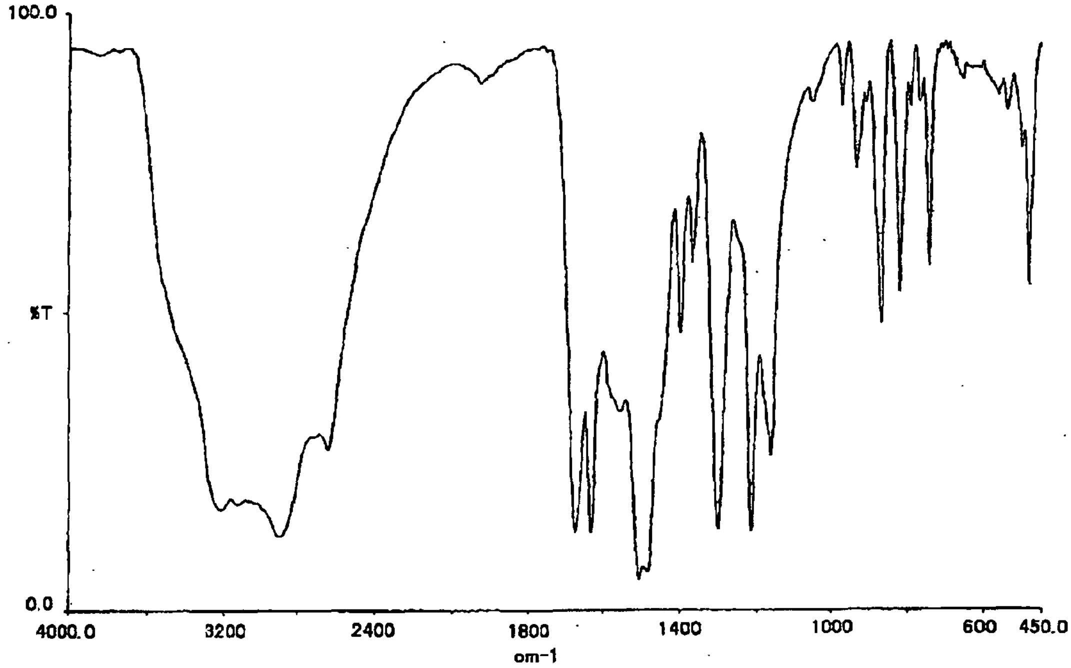

European Patent Office

# EUROPEAN SEARCH REPORT

Application Number
EP 05 02 4307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | R. ADAMS ET AL: "Structure of Gossypol. XXIV. Attempts to prepare Desapogossypolone tetramethyl ether" J.AM.CHEM.SOC., vol. 63, 1941, pages 528-534, XP002365467 * pages 529,532; compound XI * * page 535, right-hand column * | 2,3,5-7 | C07C243/38 C08K5/25 |
| X | EP 0 303 286 A (E.R. SQUIBB & SONS, INC) 15 February 1989 (1989-02-15) * page 35; compound C) * | 2 | |
| X | SUNG H-H ET AL: "EFFECT OF SOLAR SUBSTITUENTS ON THE PROPERTIES OF 1,3,4-OXADIAZOLE-BASED LIQUID CRYSTALLINE MATERIALS CONTAINING ASYMMETRIC CORES" LIQUID CRYSTALS, TAYLOR AND FRANCIS, ABINGDON, GB, vol. 31, no. 6, June 2004 (2004-06), pages 831-841, XP001195469 ISSN: 0267-8292 * page 836; compound 3A * | 1,4-7 | TECHNICAL FIELDS SEARCHED (IPC) |
| X | DATABASE CA [Online] CHEMICAL ABSTRACTS SERVICE, COLUMBUS, OHIO, US; SELIGMAN, ARNOLD M. ET AL: "A new reagent for the histochemical demonstration of active carbonyl groups. The preparation of 2-hydroxynaphthalenecarboxylic and sulfonic acid hydrazides" XP002365882 retrieved from STN Database accession no. 1950:3044 * abstract * & ENDOCRINOLOGY , 44, 584-7 CODEN: ENDOAO; ISSN: 0013-7227, 1949, | 1 | C07C |

-/--

The present search report has been drawn up for all claims

| Place of search | Date of completion of the search | Examiner |
|---|---|---|
| Munich | 3 February 2006 | Österle, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 05 02 4307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | W.H. LINNELL: "A synthesis of Indane-1-carboxylic acids"<br>J.CHEM.SOC., 1953, pages 3257-3261, XP009060942<br>* page 3261, paragraph 2 *<br>----- | 1,3,4,6, 7 | |
| D,A | US 5 534 569 A (ETOH ET AL)<br>9 July 1996 (1996-07-09)<br>* the whole document *<br>----- | 1-7 | |
| D,A | PATENT ABSTRACTS OF JAPAN<br>vol. 1997, no. 07,<br>31 July 1997 (1997-07-31)<br>& JP 09 067466 A (OTSUKA CHEM CO LTD),<br>11 March 1997 (1997-03-11)<br>* abstract *<br>----- | 1-7 | |
| | | | TECHNICAL FIELDS SEARCHED (IPC) |

The present search report has been drawn up for all claims

| Place of search | Date of completion of the search | Examiner |
|---|---|---|
| Munich | 3 February 2006 | Österle, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 02 4307

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2006

| Patent document cited in search report | Publication date | Patent family member(s) | Publication date |
|---|---|---|---|
| EP 0303286 A | 15-02-1989 | AU 2091988 A | 16-02-1989 |
| | | DK 454188 A | 14-02-1989 |
| | | HU 48235 A2 | 29-05-1989 |
| | | JP 1096181 A | 14-04-1989 |
| | | NZ 225821 A | 27-11-1990 |
| | | US 4777252 A | 11-10-1988 |
| | | ZA 8805998 A | 26-04-1989 |
| US 5534569 A | 09-07-1996 | NONE | |
| JP 09067466 A | 11-03-1997 | JP 3491111 B2 | 26-01-2004 |

EPO FORM P0459

For more details about this annex : see Official Journal of the European Patent Office, No. 12/82